# EUROPEAN PATENT APPLICATION

(11) **EP 4 477 247 A1**
(43) Date of publication of application: **18.12.2024**
(21) Application number: 24180084.6
(22) Date of filing: 05.06.2024
(51) Int. Cl.: A61M 39/06, A61B 17/34, A61F 2/24, A61N 1/375, A61M 25/06, A61M 25/01

(54) **VALVE BYPASS TOOL FOR AN IMPLANTABLE MEDICAL DEVICE DELIVERY SYSTEM AND METHOD OF IMPLANTING A MEDICAL DEVICE**

(30) Priority: 16.06.2023 US 202363508609 P; 30.05.2024 US 202418678782
(71) Applicant: Pacesetter, Inc., Sylmar, CA 91342 (US)
(72) Inventor: Arnar, Bernhard, Sylmar, CA 91342 (US); Weber, Adam, Sylmar, CA 91342 (US); Sacha, Michael, Sylmar, CA 91342 (US)
(74) Representative: Barker Brettell Sweden AB

(57) **Abstract**

A valve bypass tool (200, 300, 400, 500, 600, 700) for an implantable medical device (IMD) delivery system (100) includes a back panel (202) and a tube (204) connected to the back panel (202) and extending from the back panel (202) to a distal end (208) of the tube (204). The back panel (202) defines an inlet opening (212). The tube (204) is cylindrical and the distal end (208) of the tube (204) is configured to dilate a seal of an access introducer (110). The tube (204) defines a channel (210) therethrough that aligns with and is open to the inlet opening (212) in the back panel (202). The inlet opening (212) and the channel (210) of the tube (204) are sized to receive an IMD (10, 152) therethrough.

## Description

### TECHNICAL FIELD

Embodiments of the present disclosure generally relate to tools for delivering implantable medical devices (IMDs) into a subject. In one or more embodiments, the IMDs delivered by the tools are leadless.

### BACKGROUND

Delivery systems are used to implant IMDs within subjects. The IMD may function to monitor cardiac activity of the subject, provide electrotherapy to cardiac tissue, and/or the like. The delivery systems may include guide catheters that transport the IMD, or a lead thereof, through an access introducer into a subject. The access introducer may penetrate an incision in the subject, such that the catheter and IMD enter the subject through the access introducer. The guide catheter may navigate the venous system and/or cardiac anatomy to position the IMD, or lead thereof, at a target anatomical location. Upon placement of the IMD, or lead thereof, at the target location, the guide catheter is withdrawn from the subject.

The access introducer typically includes a hemostasis valve with a seal to block leakage of blood and other organic fluid out of an inlet opening of the access introducer through which the IMD, or lead thereof, and the guide catheter enters the access introducer. It may be difficult to advance the distal end of the guide catheter with the IMD, or lead thereof, through the hemostasis seal. For example, the hemostasis seal may be more rigid and/or stiff than the distal end of the guide catheter. Furthermore, forcing the distal end against the seal may damage one or more components of the delivery system that are relatively delicate, such as a mechanical fixation element of the IMD or a protective sleeve that covers the mechanical fixation element. Some delivery systems utilize a discrete valve bypass tool for dilating the seal of the hemostasis valve, rather than use the guide catheter or the IMD held by the guide catheter.

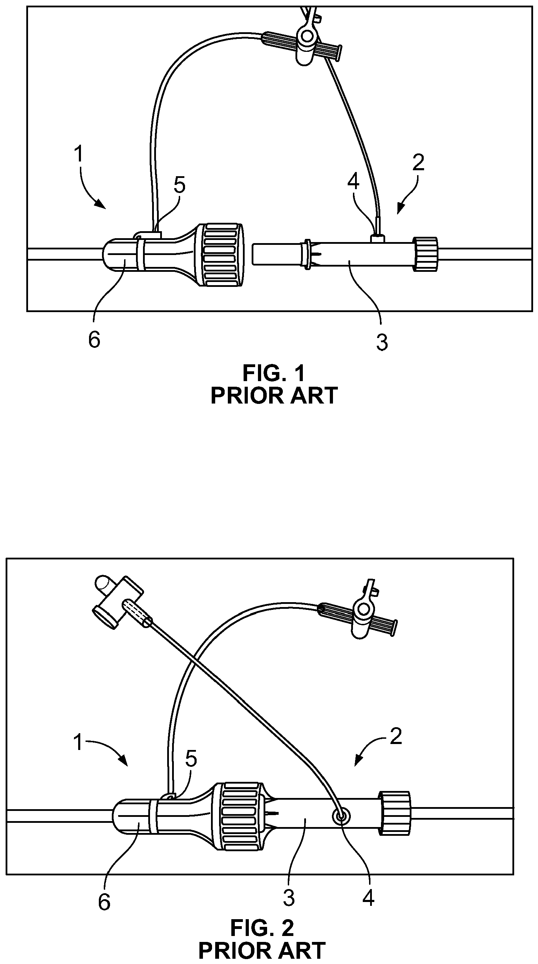

Known valve bypass tools and delivery systems that contain valve bypass tools have several drawbacks. The valve bypass tool may be relatively bulky and complex, and may occupy valuable length of the delivery system. Figure 1 illustrates an access introducer 1 next to a known valve bypass tool 2. The valve bypass tool 2 has an elongated housing 3 that contains an integrated seal. The valve bypass tool 2 also includes a side port 4 to permit flushing saline through the valve bypass tool 2 for removing air bubbles. Figure 2 illustrates the valve bypass tool 2 coupled to the access introducer 1. The elongated housing 3 may lock onto the access introducer 1 and remain coupled to the access introducer 1 throughout the use of the delivery system, even though the valve bypass tool 2 is only needed for dilating the seal of the access introducer 1 to insert and extract the IMD. The combined assembly includes multiple side ports 4, 5, multiple elongated housings 3, 6, multiple seals, and has significant length. As such, the assembly is relatively bulky and complex. Considering that the valve bypass tool 2 is only utilized during insertion and withdrawal of the delivery system relative to the access introducer 1, the presence of the relatively bulky valve bypass tool 2 throughout the implant and/or extraction procedure is relatively obtrusive.

A need remains for a valve bypass tool of a delivery system that avoids at least some of the issues with known valve bypass tools. For example, a need remains for valve bypass tools that may occupy less length and may be less complex than known valve bypass tools.

### SUMMARY

The present invention is defined in the independent claim. Further embodiments of the invention are defined in the dependent claims.

In accordance with an embodiment, a valve bypass tool for an implantable medical device (IMD) delivery system is provided. The valve bypass tool includes a back panel and a tube connected to the back panel and extending from the back panel to a distal end of the tube. The back panel defining an inlet opening. The tube is cylindrical, and the distal end of the tube is configured to dilate a seal of an access introducer. The tube defines a channel therethrough that aligns with and is open to the inlet opening in the back panel. The inlet opening and the channel of the tube are sized to receive an IMD therethrough.

Optionally, the inlet opening and the channel of the tube may be sized to receive a leadless IMD as the IMD.

Optionally, a diameter of the channel of the tube may be 15-35 F (5-11.67 mm).

Optionally, the back panel may be planar, and may include a secondary seal configured to seal around a guide catheter inserted through the valve bypass tool.

Optionally, the back panel and the tube are defined by at least a first section and a second section. The first section and the second section may be selectively movable relative to each other to define a closed state of the valve bypass tool and an open state of the valve bypass tool. In the open state, a first curved member of the first section may be spaced apart from a second curved member of the second section to define at least one gap along a length of the tube.

Optionally, the at least one gap in the open state may be larger than a diameter of a guide catheter inserted through the valve bypass tool.

Optionally, the first section may be connected to the second section via a living hinge, and the first and second sections may be pivotable about the living hinge.

Optionally, the living hinge may extend along the length of the tube and connect the first curved member to the second curved member.

Optionally, the back panel and the tube are defined by at least a first section and a second section, and the first section is coupled to the second section via a mechanical hinge.

Optionally, the first and second sections may be pivotable about the mechanical hinge.

Optionally, the back panel may be defined by at least a first flange member of the first section and a second flange member of the second section. The mechanical hinge may be secured to the first flange member and the second flange member.

Optionally, the mechanical hinge may include a pin that projects through a first ear of the first flange member and a second ear of the second flange member.

Optionally, the back panel and the tube are defined by at least a first section and a second section, and the first and second sections are movable relative to one another along a mating axis to transition between the open state and the closed state.

Optionally, the first section may include at least a first pin, and the second section may include at least a first pinhole that is sized and positioned to align with the first pin. The first pin may be received within the first pinhole as the first and second sections are moved along the mating axis to the closed state.

Optionally, the first section includes a first front flange and a first back flange extending from the first curved member. The second section may include a second front flange and a second back flange extending from the second curved member. The first front flange may overlap and contact the second back flange, and the first back flange may overlap and contact the second front flange, as the first and second sections are moved along the mating axis to the closed state.

In an embodiment, a method for assembling an implantable medical device (IMD) delivery system is provided. The method includes inserting a valve bypass tool (VBT) into a first inlet opening of an access introducer. The VBT may include a back panel and a tube connected to the back panel. The tube may extend from the back panel to a distal end of the tube. The tube may be cylindrical and may define a channel therethrough that aligns with and is open to a second inlet opening defined through the back panel. Inserting the VBT may include advancing the tube into the first inlet opening such that the distal end of the tube dilates a seal of the access introducer. The method may include inserting an IMD and a guide catheter through the second inlet opening and the channel of the VBT while the tube is within the access introducer such that the IMD and the guide catheter pass beyond the seal of the access introducer.

Optionally, inserting the IMD and the guide catheter may include inserting a leadless IMD through the second inlet opening and the channel of the VBT.

Optionally, the method may include removing the VBT from around the guide catheter while a portion of the guide catheter is located within the access introducer.

Optionally, the back panel and the tube of the VBT may be defined by at least a first section and a second section such that the tube is defined by at least a first curved member of the first section and a second curved member of the second section. Removing the VBT may include pivoting the first section of the VBT relative to the second section of the VBT about a hinge to cause at least one gap along a length of the tube between the first curved member and the second curved member.

Optionally, the hinge may be a living hinge that extends along the length of the tube and connects the first curved member to the second curved member.

Optionally, the back panel and the tube of the VBT may be defined by at least a first section and a second section. The tube may be defined by at least a first curved member of the first section and a second curved member of the second section. Removing the VBT may include separating the first and second sections along a mating axis to disconnect the first section from the second section.

### BRIEF DESCRIPTION OF THE DRAWINGS

Figure 1 illustrates an access introducer next to a known valve bypass tool.
Figure 2 illustrates the known valve bypass tool coupled to the access introducer.
Figure 3 illustrates a sectional view of a patient's heart and shows a leadless intra-cardiac medical device.
Figure 4 illustrates a delivery system according to an embodiment.
Figure 5 illustrates a protective sleeve surrounding an IMD according to an embodiment.
Figure 6 illustrates the protective sleeve of Figure 5 retracted relative to the IMD.
Figure 7 is a perspective view of a valve bypass tool (VBT) in a closed state according to a first embodiment.
Figure 8a is a front view of the VBT shown in Figure 7.
Figure 8b is an enlarged view of a portion of the VBT shown in Figure 8a.
Figure 9 is a front view of the VBT of Figure 7 in an open state.
Figure 10 is an exploded view of an access introducer, the VBT, the protective sleeve, and a guide catheter of the delivery system according to an embodiment.
Figure 11 is a perspective view of a VBT in the closed state according to a second embodiment.
Figure 12 is a perspective view of the VBT of Figure 11 in the open state.
Figure 13 is a cross-sectional view of the VBT of Figure 11.
Figure 14 is a perspective view of a VBT in the closed state according to a third embodiment.
Figure 15 is a perspective view of the VBT of Figure 14 in the open state.
Figure 16 is a perspective view of a VBT in the closed state according to a fourth embodiment.
Figure 17 is a perspective view of the VBT of Figure 16 in the open state.
Figure 18 is a perspective view of a VBT in the open state according to a fifth embodiment.
Figure 19 is a perspective view of the VBT of Figure 18 in the closed state.
Figure 20 is a front perspective view of a VBT according to a sixth embodiment.
Figure 21 is a back perspective view of the VBT of Figure 20.
Figure 22 is a flow chart of a method for assembling an IMD delivery system according to an embodiment.

### DETAILED DESCRIPTION

It will be readily understood that the components of the embodiments as generally described and illustrated in the figures herein, may be arranged and designed in a wide variety of different configurations in addition to the described example embodiments. Thus, the following more detailed description of the example embodiments, as represented in the figures, is not intended to limit the scope of the embodiments, as claimed, but is merely representative of example embodiments.

Reference throughout this specification to "one embodiment" or "an embodiment" (or the like) means that a particular feature, structure, or characteristic described in connection with the embodiment is included in at least one embodiment. Thus, appearances of the phrases "in one embodiment" or "in an embodiment" or the like in various places throughout this specification are not necessarily all referring to the same embodiment.

Furthermore, the described features, structures, or characteristics may be combined in any suitable manner in one or more embodiments. In the following description, numerous specific details are provided to give a thorough understanding of embodiments. One skilled in the relevant art will recognize, however, that the various embodiments can be practiced without one or more of the specific details, or with other methods, components, materials, etc. In other instances, well-known structures, materials, or operations are not shown or described in detail to avoid obfuscation. The following description is intended only by way of example, and simply illustrates certain example embodiments.

The methods described herein may employ structures or aspects of various embodiments (e.g., systems and/or methods) discussed herein. In various embodiments, certain operations may be omitted or added, certain operations may be combined, certain operations may be performed simultaneously, certain operations may be performed concurrently, certain operations may be split into multiple operations, certain operations may be performed in a different order, or certain operations or series of operations may be re-performed in an iterative fashion. It should be noted that, other methods may be used, in accordance with an embodiment herein. Further, wherein indicated, the methods may be fully or partially implemented by one or more processors of one or more devices or systems. While the operations of some methods may be described as performed by the processor(s) of one device, additionally, some or all of such operations may be performed by the processor(s) of another device described herein.

Embodiments may be implemented in connection with one or more implantable medical devices (IMDs). Non-limiting examples of IMDs include neurostimulator devices, implantable leadless monitoring and/or therapy devices, catheters, and/or alternative implantable medical devices. For example, the IMD may represent a cardiac monitoring device, pacemaker, cardioverter, cardiac rhythm management device, defibrillator, neurostimulator, leadless monitoring device, leadless pacemaker and the like. For example, the IMD may include one or more structural and/or functional aspects of the device(s) described in U.S. Patent 9,333,351 "Neurostimulation Method And System To Treat Apnea" and U.S. Patent 9,044,610 "System And Methods For Providing A Distributed Virtual Stimulation Cathode For Use With An Implantable Neurostimulation System".

In one or more embodiments, the IMD may be a leadless implantable medical device (LIMD) that include one or more structural and/or functional aspects of the device(s) described in U.S. Patent 9,216,285 "Leadless Implantable Medical Device Having Removable And Fixed Components" and U.S. Patent 8,831,747 "Leadless Neurostimulation Device And Method Including The Same". Additionally, or alternatively, the IMD may include one or more structural and/or functional aspects of the device(s) described in U.S. Patent 8,391,980 "Method And System For Identifying A Potential Lead Failure In An Implantable Medical Device" and U.S. Patent 9,232,485 "System And Method For Selectively Communicating With An Implantable Medical Device".

Additionally or alternatively, the IMD may be a subcutaneous IMD (e.g., a S-ICD) that includes one or more structural and/or functional aspects of the device(s) described in U.S. Publication No.: 2023/0381500, titled "Method And Implantable Medical Device For Reducing Defibrillation Impedance"; U.S. Patent No.: 10,765,860, titled "Subcutaneous Implantation Medical Device With Multiple Parasternal-Anterior Electrodes"; U.S. Patent No.: 10,722,704, titled "Implantable Medical Systems And Methods Including Pulse Generators And Leads"; U.S. Patent No.: 11,045,643, titled "Single Site Implantation Methods For Medical Devices Having Multiple Leads". Further, one or more combinations of IMDs may be utilized from the above mentioned patents and applications in accordance with embodiments herein.

The term "leadless" shall mean an absence of transvenous and/or subcutaneous electrically-conductive leads that would otherwise traverse vessels or other anatomy inside or outside of an intra-cardiac space.

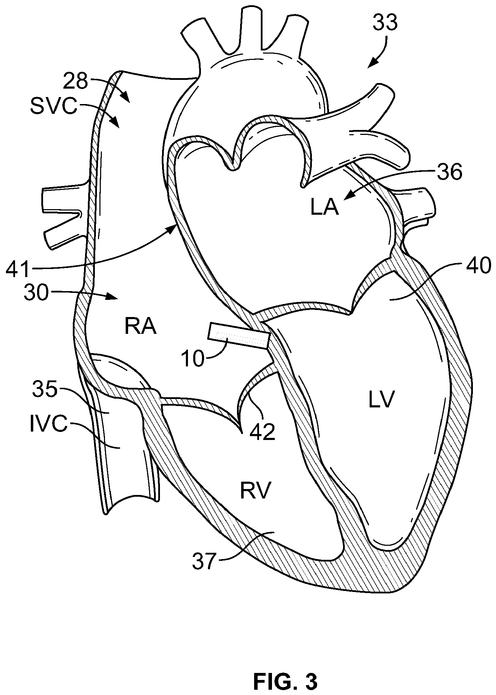

Figure 3 illustrates a sectional view of a patient's heart 33 and shows a leadless intra-cardiac medical device 10. The leadless implantable medical device 10 has been placed through the superior vena cava 28 into the right atrium (RA) 30 of the heart 33. Figure 3 also shows the inferior vena cava 35, the left atrium (LA) 36, the right ventricle (RV) 37, the left ventricle (LV) 40, the atrial septum 41, the ventricular vestibule (VV), the right atrial appendage (RAA), and the tricuspid valve 42. The atrial septum 41 divides the two atria 30, 36. The tricuspid valve 42 is between the right atrium 30 and right ventricle 37. The term "septum" generally refers to any portion of the heart separating two chambers (e.g., RA to LA, RV to LV). The leadless implantable medical device (LIMD) 10 may represent a pacemaker (e.g., a leadless pacemaker). The leadless pacemaker may function in a mode that senses in two chambers, paces in two chambers, and inhibits pacing in either chamber based on intrinsic events sensed in one or both of the chambers. Alternatively, the LIMD 10 may represent a cardiac resynchronization device, a cardioverter, a defibrillator, or the like. The LIMD 10 may include a housing configured to be implanted entirely within a single local chamber of the heart. For example, the LIMD 10 may be implanted entirely and solely within the RA or entirely and solely within the RV. Optionally, the LIMD 10 may be implanted entirely and solely within the LA or the LV through more invasive implant methods.

The LIMD 10 optionally may be implanted in an area near different regions of tissue that follow the conductive pattern of different chambers of the heart. For example, the LIMD 10 may be implanted such that at least one electrode on the housing of the LIMD 10 engages tissue that is part of the conductive network of the one chamber, while at least one other electrode projects from the housing into tissue that is part of the conductive network of another chamber. For example, the LIMD 10 may be implanted within or near the triangle of Koch in an area adjacent the ventricular vestibule. The conductive network of the tissue in the ventricular vestibule follows the conductive pattern of the right ventricle. Therefore, one or more electrodes of the LIMD 10 may be electrically coupled to the conductive network of the right atrium, and one or more other electrodes may extend diagonally to electrically connect to the conductive network of the right ventricle (e.g., the ventricular vestibule). Optionally, the LIMD 10 may be positioned with the housing located against the RA wall above the mitral valve, but with a distal electrode that projects into the septum to ventricular tissue of the right or left ventricle.

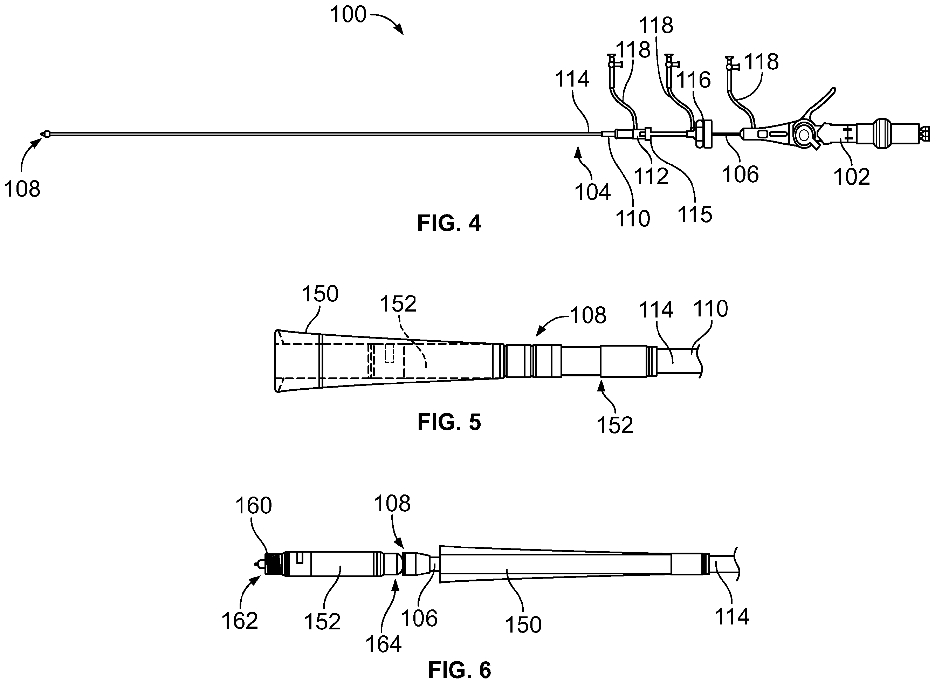

Figure 4 illustrates a delivery system 100 according to an embodiment. The delivery system 100 includes a handle 102 and an elongated delivery assembly 104 coupled to the handle 102. The handle 102 may be manipulated by an operator to control the components of the delivery assembly 104. The delivery assembly 104 includes a guide catheter 106 that extends from the handle 102 to a distal end 108 of the guide catheter 106. The distal end 108 of the guide catheter 106 may define a distal end of the delivery system 100. The guide catheter 106 may hold and transport an IMD through the body of the subject to a target implant location. For example, the distal end 108 of the guide catheter 106 may couple to the IMD during an implant procedure, and may uncouple from the IMD after the IMD is positioned at the target location. Furthermore, the distal end 108 may couple to the IMD during an extraction procedure to remove the IMD from the subject.

The delivery assembly 104 may include an access introducer 110 which has a housing 112 and a tube 114 extending from the housing 112. The tube 114 defines a lumen. The guide catheter 106 and the IMD held by the guide catheter 106 may be received within the lumen during the implant procedure. During implant and extraction, the access introducer 110 may penetrate an incision within the subject such that a first or distal portion of the tube 114 is disposed within the anatomy of the subject and a second or proximal portion of the tube 114 as well as the housing 112 are disposed outside of the anatomy. The guide catheter 106 may be inserted through an inlet opening in the housing 112, outside of the subject, into the lumen of the tube 114 and then advanced along the length of the tube 114 towards the target implant location. The tube 114 optionally may extend at least a majority of the length of the delivery assembly 104 that extends from the handle 102.

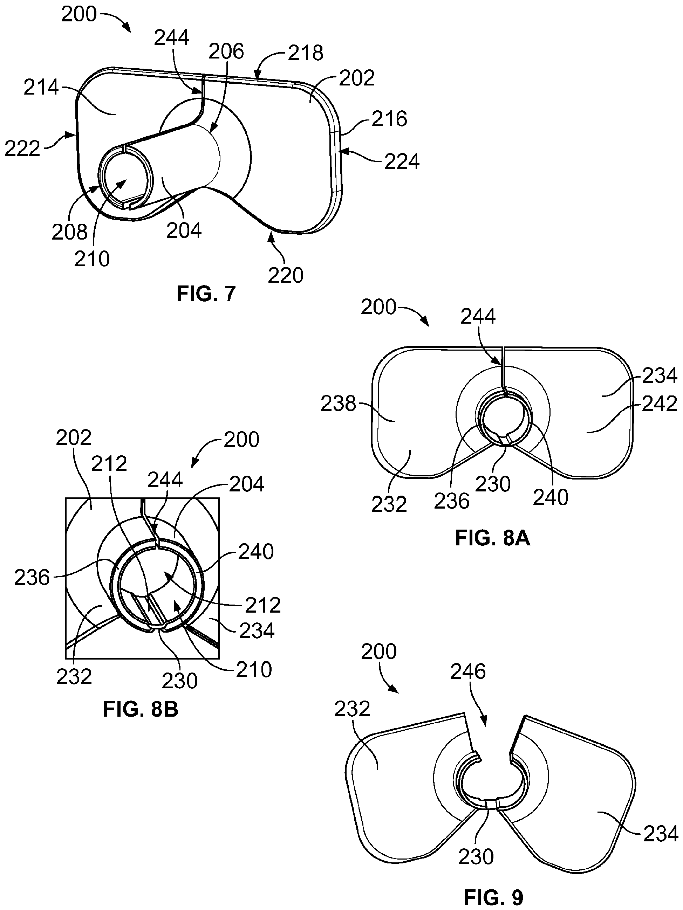

The housing 112 of the access introducer 110 may include a hemostasis seal. The delivery system 100 includes a valve bypass tool 115 that selectively dilates the hemostasis seal of the access introducer 110 to permit the guide catheter 106 and the IMD to move through the seal. The valve bypass tool 115 is described herein in more detail with reference to the embodiments shown in Figures 7 through 21. In one or more embodiments, the valve bypass tool (VBT) 115 can transition between a closed state and an open state. In the closed state, the VBT 115 defines a cylindrical tube that is sized to fit within an inlet opening of the housing 112 of the access introducer 110. The cylindrical tube may dilate the hemostasis seal when the VBT 115 is pressed towards the housing 112. In the open state, the VBT 115 does not define the cylindrical tube. For example, the VBT 115 may include a first curved member and a second curved member. The closed state, the first and second curved members are positioned relative to one another to each define a respective portion of the circumference of the cylindrical tube. To transition from the closed state to the open state, at least one of the first curved member or the second curved member may move away from the other curved member. In one or more embodiments, the VBT 115 in the open state may be removable from the access introducer 110 and/or the guide catheter 106. As such, the VBT 115 can be removed from the delivery assembly 104 when not needed, which can free up space along the length of the delivery assembly 104.

The delivery assembly 104 may include a catheter locking hub 116 disposed between the access introducer 110 and the handle 102 along the length of the delivery system 100. The catheter locking hub 116 may be actuated to secure a protective sleeve around the IMD carried by the guide catheter 106.

Figure 5 illustrates a protective sleeve 150 surrounding an IMD 152 according to an embodiment. Figure 5 may show the protective sleeve 150 and the IMD 152 in a delivery configuration that corresponds to the positioning of the components during the implant procedure as the IMD 152 is advanced towards the target implant location. The protective sleeve 150 is illustrated as partially see-through to show the IMD 152. The protective sleeve 150 and the IMD 152 may be located at the distal end 108 of the guide catheter 106. For example, the IMD 152 may be secured to the distal end 108 and project beyond the distal end 108. The protective sleeve 150 surrounds the IMD 152 along the entire length of the IMD 152. Optionally, the protective sleeve 150 and the IMD 152 may protrude beyond a distal end 154 of the tube 114 of the access introducer 110.

With continued reference to Figures 4 and 5, the catheter locking hub 116 may tighten the protective sleeve 150 to the IMD 152 and/or to the catheter 106 to prohibit movement of the protective sleeve 150 relative to the IMD 152 which could potentially expose a mechanical fixation element of the IMD 152 during implant. Locking the protective sleeve 150 relative to the IMD 152 may avoid potential damage to the mechanical fixation element and/or harm to soft tissue of the subject caused by the mechanical fixation element as the IMD 152 is advanced through the anatomy.

Figure 6 illustrates the protective sleeve 150 of Figure 5 retracted relative to the IMD 152. The protective sleeve 150 does not surround the IMD 152 in the illustrated configuration. The IMD 152 may remain coupled to the distal end 108 of the guide catheter 106, while the protective sleeve 150 and the tube 114 are in retracted positions relative to the configuration shown in Figure 5.

The IMD 152 includes a mechanical fixation element 160 at a distal end 162 of the IMD 152. A proximal end 164 of the IMD 152, opposite the distal end 162, may be removably secured to the guide catheter 106. The mechanical fixation element 160 may be a helical screw. The IMD 152 may anchor to cardiac tissue of the subject at the target implant location by controlling the guide catheter 106, via the handle 102, to rotate the IMD 152 while the helical screw is pressed against a cardiac wall, causing the helical screw to embed into the cardiac wall. The mechanical fixation element 160 may be other than a helical screw in other embodiments such as deflectable anchoring tabs. When the IMD 152 is not at the target implant location, the protective sleeve 150 may surround the IMD 152, as shown in Figure 5, to function as an intermediary or buffer which is disposed between mechanical fixation element 160 and the subject tissue.

In the illustrated embodiment, the IMD 152 is leadless. For example, the IMD 152 may be the LIMD 10 shown in Figure 3. The IMD 152 may represent a leadless pacemaker. The embodiments of the delivery system 100 and the valve bypass tool thereof described herein may not be limited to leadless IMD applications.

Referring now back to Figure 4, the access introducer 110, the catheter locking hub 116, and/or the handle 102 optionally may include a side port 118. In Figure 4, all three components include a respective side port 118. The side ports 118 include flexible hoses or tubes that are fluidly connected to interior channels of the components. The side ports 118 permit flushing the interior channels with saline or another fluid to remove air bubbles.

The delivery system 100 in other embodiments may include additional components than the components shown in Figure 4 and/or may lack one or more of the components shown in Figure 4.

Figure 7 is a perspective view of a valve bypass tool (VBT) 200 in a closed state according to a first embodiment. The VBT 200 may represent the VBT 115 of the delivery system 100 shown in Figure 4. The VBT 200 includes a back panel 202 and a tube 204 that projects from the back panel 202. A proximal end 206 of the tube 204 is attached to the back panel 202. The tube 204 is cantilevered from the back panel 202 such that a distal end 208 of the tube 204 is spaced apart from the back panel 202. The tube 204 is hollow and defines a channel 210 through the length of the tube 204. The channel 210 is open at the proximal and distal ends 206, 208 of the tube 204. The channel 210 aligns with and is fluidly connected to an inlet opening 212 (shown in Figures 8a and 8b) of the back panel 202. In an embodiment, the tube 204 is cylindrical, such that the tube 204 has a circular cross-sectional shape. In other embodiments, the tube 204 may have a different cross-sectional shape, such as oblong, rectangular, or the like.

The back panel 202 has a front side 214 and a back side 216 opposite the front side 214. The front side 214 and/or the back side 216 may be relatively flat and planar. A depth or thickness of the back panel 202 is defined between the front and back sides 214, 216. The back panel 202 has a height (or length) from a first end 218 to a second end 220 that is opposite the first end 218. The back panel 202 has a width from a third end 222 to a fourth end 224 that is opposite the third end 222. In an embodiment, the back panel 202 is relatively thin such that the depth or thickness of the back panel 202 is substantially less than the height and width of the back panel 202. For example, the height and width may be several multiples of the depth dimension.

Figure 8a is a front view of the VBT 200 shown in Figure 7. Figure 8b is an enlarged view of a portion of the VBT 200 shown in Figure 8a. In the illustrated embodiment, the VBT 200 includes a living hinge 230 that connects a first section 232 of the VBT 200 to a second section 234 of the VBT 200. The first and second sections 232, 234 define the tube 204 and the back panel 202. For example, the first section 232 includes a first curved member 236 and a first flange member 238. The second section 234 includes a second curved member 240 and a second flange member 242. The tube 204 is defined by the first and second curved members 236 and 240 and the living hinge 230. For example, the living hinge 230 extends along the length of the tube 204 and connects the first curved member 236 to the second curved member 240. The first and second flange members 238, 242 define the back panel 202.

The living hinge 230 may be a relatively thin, flexible layer. The living hinge 230 may be the only component that mechanically connects the first section 232 to the second section 234. For example, the living hinge 230 extends along a length of an interface between the first section 232 and the second section 234. When the VBT 200 is in the closed position as shown in Figures 7, 8a, and 8b, the first and second sections 232, 234 define a seam 244 (e.g., slit, gap, etc.) along the lengths of the interface outside of the living hinge 230. For example, when the VBT 200 is oriented as shown in Figures 8a and 8b, the seam 244 extends the full length of the tube 204, and continues along the back panel 202 to the first (e.g., top) end 218. The living hinge 230 flexes or articulates to enable the first section 232 and/or the second section 234 to pivot relative to one another, at the living hinge 230, which changes the size of the seam 244. In the closed state, edges of the first and second sections 232, 234 may physically contact each other at the seam 244, or may be separated by a narrow slit.

Figure 9 is a front view of the VBT 200 of Figure 7 in an open state. In the open state, the living hinge 230 is flexed to permit that first and second sections 232, 234 to be spaced apart a greater distance from each other at the interface, forming a large gap 246. The large gap 246 extends along both the tube 204 and the back panel 202. The transition from the closed state to the open state involves pivoting the first and second sections 232, 234 at the living hinge 230 to convert the narrow seam 244 at the interface to the large gap 246. Optionally, the VBT 200 may be selectively pivoted to cause a larger gap than the gap 246 shown in Figure 9. The VBT 200 may be configured in the open state in order to install the VBT 200 and/or remove the VBT 200 from the delivery system 100, as described herein. For example, in the open state the channel 210 defined by the tube 204 no longer has a closed cross-sectional perimeter, so the VBT 200 can be removed from around the guide catheter 106 or another component that is present within the channel 210 of the tube 204.

In an embodiment, the living hinge 230 is integrally connected with the first and second curved sections 236, 240 that define the tube 204. For example, the living hinge 230 may be an extension of the first and second curved sections 236, 240 that is thinner, and therefore less rigid, than the first and second curved sections 236, 240. In an example, the VBT 200 may have a unitary, monolithic body that is composed of a single material. The VBT 200 may be formed into the shape shown in Figures 7 through 9 via a molding process, a welding process, additive manufacturing, or the like. In a first example, the VBT 200 may be composed of a plastic material and formed via a molding process. In a second example, the VBT 200 may be composed of a metal material that is stamped and formed into shape.

In another embodiment, the living hinge 230 may be a discrete component that is secured to the first and second curved sections 236, 240 during manufacturing via bonding, molding, welding, or the like. Optionally, the first and second curved sections 236, 240 of the tube 204 may be discrete components from the first and second flanges 238, 242 of the back panel 202. For example, the curved sections 236, 240 may be composed of a metal material, and the flanges 238, 242 may be composed of a plastic material.

The living hinge VBT 200 shown in Figures 7 through 9 may have the benefits of being relatively small and uncomplex. The VBT 200 is also removable to avoid the obtrusiveness of the VBT 200 on the delivery system 100 when not needed.

The diameter of the channel 210 of the tube 204 may be sufficiently large to accommodate entire IMDs, such as the LIMD 152 shown in Figure 6. For example, the diameter of the channel 210 of the tube 210 may be 15-35 F (5-11.67 mm). The access introducer 110 may also be sized to accommodate this relatively large payload. As a consequence, the hemostasis seal of the access introducer 110 may require significantly more force to dilate than smaller seals that only accommodate an electrical lead. Electrical leads may be around 6 F (2 mm) in diameter, so the tube 204 of the VBT 200 has to withstand significantly more force than conventional VBTs used to advance leads into access introducers. In an embodiment, the tube 204 of the VBT 200 achieves significant structural support and rigidity based on the shape of the tube 204. For example, the tube 204 may have a cylindrical shape, which may provide sufficient support and rigidity to dilate the relatively large seal of the access introducer 110. In an embodiment in which the curved members 236, 240 that define the tube 204 are composed of a plastic material, the cylindrical shape may provide sufficient support and rigidity to avoid the tube 204 bending and/or breaking during an attempt to dilate the seal. Furthermore, the edge of the first curved member 236 may abut the edge of the second curved member 240 when in the closed state, which establishes full 360 degree mechanical support even in the presence of the seam 244. In another embodiment in which the edges of the curved members 236, 240 do not contact one another in the closed state, the VBT 200 may be designed such that the size of the seam 244 is no greater than a threshold size, such as 10 degrees (e.g., the curved sections 236, 240 define at least 350 degrees), to retain the support and rigidity necessary to dilate the seal.

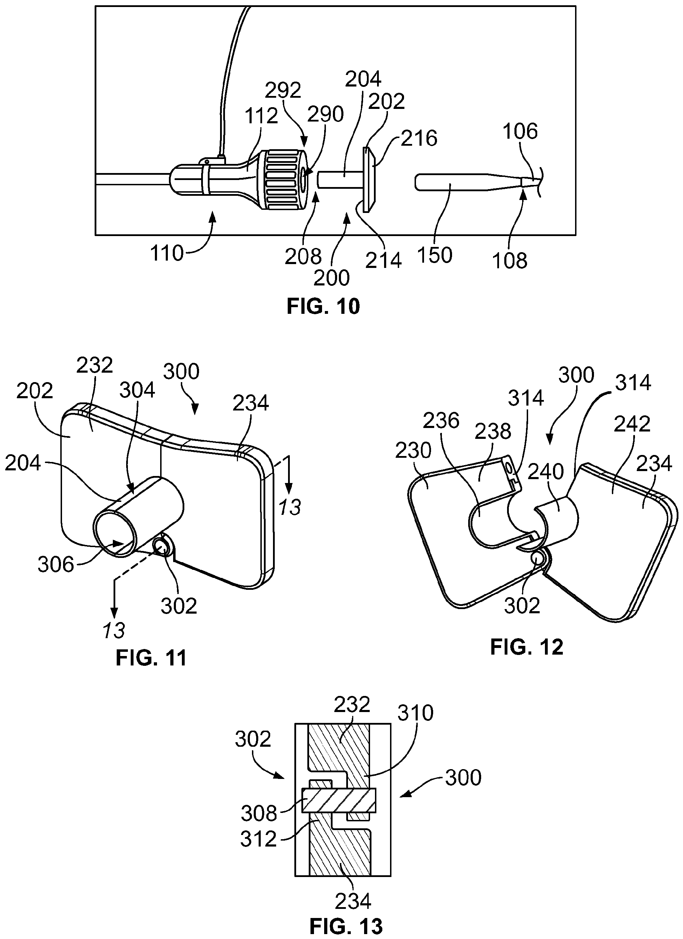

Figure 10 is an exploded view of the access introducer 110, the VBT 200, the protective sleeve 150, and the guide catheter 106 of the delivery system 100 according to an embodiment. The protective sleeve 150 may be surrounding an IMD which is coupled to the distal end 108 of the guide catheter 106. The following series of steps describe a process for introducing the protective sleeve 150 and the guide catheter 106 into the access introducer 110. These steps may occur during an implant procedure to place an IMD at the target implant location within the anatomy of a subject, as well as during an extraction procedure to remove and withdraw an IMD from the subject's anatomy.

The housing 112 of the access introducer 110 defines an inlet opening 290 at a proximal end 292 of the housing 112. The first step in the procedure is to insert the tube 204 of the VBT 200 into the inlet opening 290. The VBT 200 and the housing 112 are moved towards each other such that the back panel 202 of the VBT 200 approaches the proximal end 292 of the housing 112. For example, an operator may press against the back side 216 of the back panel 202. This movement causes the distal end 208 of the tube 204 to press against and dilate the seal of the hemostasis valve within the housing 112. Optionally, the VBT 200 may be advanced until the front side 214 of the back panel 202 abuts the proximal end 292 of the housing 112. The VBT 200 may be in the closed state in order for the tube 204 to fit within the inlet opening 290. Once inserted into the housing 112, the housing 112 may retain the VBT 200 in the closed state.

With the VBT 200 coupled to the housing 112 as described, the guide catheter 106 and attached components (e.g., protective sleeve 150, IMD 152 (shown in Figure 6), etc.) are advanced through the VBT 200. For example, the guide catheter 106 and attached components move through the inlet opening 212 (shown in Figure 8b) of the back panel 202 and the channel 210 (shown in Figure 8b) of the tube 204. The guide catheter 106 and attached components may pass beyond the seal of the hemostasis valve of the housing 112 while inside the tube 202 of the VBT 200.

After the attached components and the distal end 108 of the guide catheter 106 are moved beyond the tube 202 of the VBT 200, a segment of the guide catheter 106 is still present within the VBT 200. The VBT 200 may not be needed for a next series of steps to implant the IMD within the subject. The VBT 200 optionally may be removed from the delivery system 100. For example, an operator may grip the back panel 202 and pull the VBT 200 out of the housing 112. Then the operator may flex the VBT 200 to spread apart the VBT 200 and achieve the open state shown in Figure 9. In the open state, the operator can force the segment of the guide catheter 106 through the large gap 246 (shown in Figure 9) between the two sections 232, 234 of the VBT 200 to uncouple the VBT 200 from the guide catheter 106 and remove the VBT 200 from the delivery system 100. With the VBT 200 removed, the seal of the hemostasis valve of the access introducer 110 may seal around the guide catheter 106 without requiring a secondary seal.

Towards the end of the implant procedure and/or extraction procedure, the VBT 200 may be reinstalled to assist with removing the guide catheter 106 from the access introducer 110. This process is essentially the reverse of the process described above. The VBT 200 is spread apart to side-load onto the guide catheter 106 through the large gap 246. Then the VBT 200 is transitioned to the closed state and advanced towards the housing 112 such that the tube 204 enters the inlet opening 290. Additional advancement of the VBT 200 causes the tube 204 to dilate the seal of the hemostasis valve, relieving the force exerted on the guide catheter 106 by the seal. The released guide catheter 106 can then be pulled all the way out of the access introducer 110 and fully through the VBT 200. With the guide catheter 106 disconnected, the VBT 200 may then be removed from the access introducer 110 by pulling the back panel 202 away from the housing 112 until the tube 204 exits the inlet opening 290 of the housing 112.

Although Figure 10 refers to the VBT 200 shown in Figures 7 through 9, the same or similar steps may be performed using the removable VBTs of other embodiments described herein.

Figure 11 is a perspective view of a VBT 300 in the closed state according to a second embodiment. Figure 12 is a perspective view of the VBT 300 of Figure 11 in the open state. Figure 13 is a cross-sectional view of the VBT 300 of Figure 11. The cross-section may be taken along line 13-13 in Figure 11. The VBT 300 includes the first and second sections 232, 234 that define the tube 204 and the back panel 202, similar to the VBT 200. The first and second sections 232, 234 are pivotable relative to each other to transition the VBT 300 from the closed position shown in Figure 11 to the open position in Figure 12. The VBT 300 differs from the VBT 200 in how the first and second sections 232, 234 are connected. For example, instead of a living hinge 230, the VBT 300 has a mechanical hinge 302 that connects the first section 232 to the second section 234. The sections 232, 234 pivot about the mechanical hinge 302.

In an embodiment, the mechanical hinge 302 is affixed to the back panel 202, unlike the living hinge 230 of the VBT 200 which extends along the tube 204. The mechanical hinge 302 may be spaced apart from the tube 204. In an example, the first and second sections 232, 234 are discrete and only coupled to one another via the hinge 302. As such, the first curved member 236 that defines a portion of the tube 204 is not directly attached or secured to the second curved member 240 that defines a remainder of the tube 204. In the closed state, as shown in Figure 11, the two curved members 236, 240 form the cylindrical tube 204. The curved members 236, 240 align at contact interfaces to define two seams 304, 306. The two seams 304, 306 may be spaced 180 degrees apart along a circumference of the tube 204. In the open state, as shown in Figure 12, the two curved members 236, 240 are physically spaced apart from each other along both seams 304, 306 (e.g., without any intervening structure).

As shown in Figure 13, the mechanical hinge 302 may include a pin 308 that extends through complementary ears 310, 312 of the first and second sections 232, 234, respectively. The pin 308 may be secured to the ears 310, 312 via a friction fit, a molding process, adhesive, a mechanical clamping function, or the like.

The VBT 300 optionally may include at least one retaining element and/or biasing element to encourage the VBT 300 to maintain and/or transition to the closed position. For example, the at least one retaining element may provide a mating function between the first and second sections 232, 234 while the VBT 300 is in the closed position. The at least one retaining element exerts a force to oppose pivoting the sections 232, 234 away from one another to transition to the open state. Examples of the at least one retaining element may include at least one magnet, at least one latch, at least one friction fit element, at least one hook, at least one dowel post, and/or the like. The at least one retaining element may be installed along, or close to, interface edges 314 of one or both of the flanges 238, 242 that define the back panel 202. The interface edges 314 extend along and define the seam 244. In a first example, a first magnet may be embedded along the interface edge 314 of the first flanges 238, and a metal element or another magnet may be embedded along the opposing interface edge 314, such that the first magnet interacts with the metal element or second magnet via magnetic attraction to oppose the VBT 300 transitioning to the open state. In another example, the mechanical hinge 302 may include a biasing element, such as a torsion spring, that biases the VBT 300 towards the closed state or towards the open state. The retaining element(s) and/or biasing element(s) are optional, such that one or more embodiments may not include any retaining element(s) or biasing element(s). For example, VBT 300 may freely transition between the closed and open states. The inlet opening 290 of the access introducer 110 may be sized slightly larger than the diameter of the tube 204 in the closed state such that the VBT 300 has to be in the closed state, or approximately in the closed state, for the tube 204 to fit into the access introducer 110. This forces the pivotable VBT 300 to adopt the cylindrical shape which provides sufficient structural support and rigidity to dilate the rigid seal of the hemostasis valve.

In an example shown in Figures 11 and 12, the first and second curved members 236, 240 may be composed of a metal material, such as stainless steel. The curved members 236, 240 may be formed by machining, casting, or the like. The back panel 202, or at least the exterior surfaces thereof, may be composed of a polymer material, such as a plastic material. For example, the polymer material may be overmolded onto the proximal ends of the curved members 236, 240 during a molding process. In another example, the polymer material may be bonded to the metallic curved members 236, 240 via an adhesive. In a third example, the back panel 202 may include a thin metal sheet that is connected to the curved members 236, 240 which define the tube 204. The thin metal sheet may be encased in the polymer material.

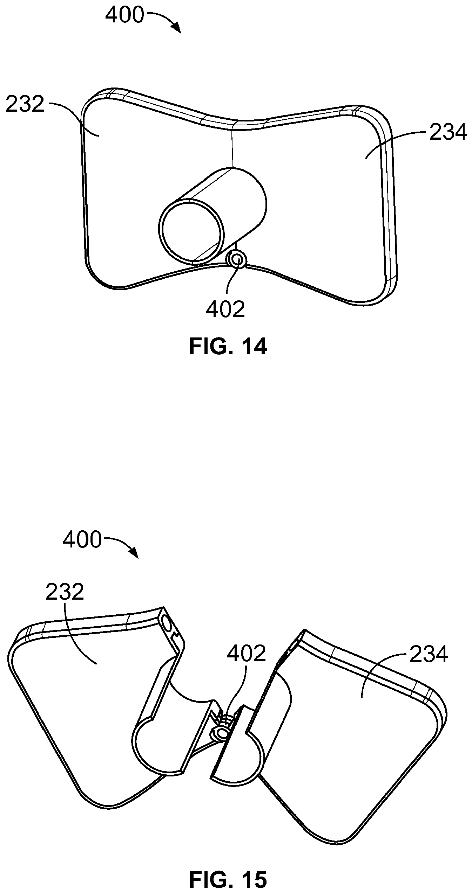

Figure 14 is a perspective view of a VBT 400 in the closed state according to a third embodiment. Figure 15 is a perspective view of the VBT 400 of Figure 14 in the open state. The VBT 400 is a removable VBT that is similar to the VBT 300 shown in Figures 11 through 13 except for the type of mechanical hinge. For example, the VBT 400 includes a mechanical hinge 402 that has a manufactured pivoting mechanism. The hinge 402 may be an off-the-shelf hinge that is separately manufactured and then installed to connect to the first and second sections 232, 234. The hinge 402 may be similar to a cabinet door hinge. The hinge 402 may perform a similar function as the mechanical hinge 302 of the VBT 300. The hinge 402 may be coupled to the sections 232, 234 via fasteners, overmolding, bonding, welding, or the like.

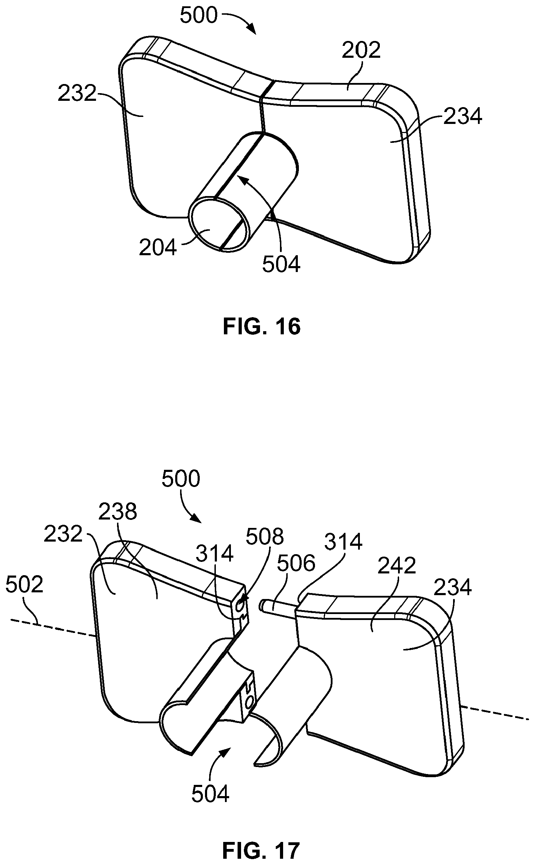

Figure 16 is a perspective view of a VBT 500 in the closed state according to a fourth embodiment. Figure 17 is a perspective view of the VBT 500 of Figure 16 in the open state. The VBT 500 may have the same or similar discrete first and second sections 232, 234 as the VBTs 200, 300, 400, such that the first and second sections 232, 234 selectively close to define the tube 204 and open to allow the VBT 500 to be removed from around the guide catheter 106. The VBT 500 differs from earlier embodiments because instead of pivoting about a pivot point, the two sections 232, 234 transition between the closed and open states by linear movement towards and away from each other along a mating axis 502. For example, to transition from the closed state shown in Figure 16 to the open state shown in Figure 17, at least one of the two sections 232, 234 is moved along the mating axis 502 in a direction away from the other section 232, 234 to separate the two sections 232, 234 at a joint 504. In the open state, the first section 232 may be physically disconnected (e.g., uncoupled) from the second section 234, such that the two components are separated in space without any linking component. In an example, the joint 504 may define a line that bisects the back panel 202 and the tube 204. The first section 232 optionally may be a replica of the second section 234, such that the first and second sections 232 are manufactured to have the same size, shape, and composition.

In an embodiment, the VBT 500 includes retaining elements and/or alignment elements for guiding the mating of the two sections 232, 234 and securing the two sections 232, 234 together at the joint 504 to achieve the closed state shown in Figure 16. In the illustrated example, the VBT 500 may include at least one pin 506 (e.g., dowel pin) or post that protrudes from the interface edge 314 of at least one of the flanges 238, 242 and is configured to be received and nested into a pinhole 508 defined in the opposing interface edge 314 of the other flange 238, 242. The pin 506 may project in a direction that is parallel to the mating axis 502. The entry of the pin 506 into the pinhole 508 may guide the coupling of the two sections 232, 234 along the mating axis 502, providing both alignment and retention via a friction fit. In an example, each of the two sections 232, 234 has one pin 506 and one pinhole 508, such that the pin 506 of the first section 232 is received in the pinhole 508 of the second section 234 and the pin 506 of the second section 234 is received in the pinhole 508 of the first section 232.

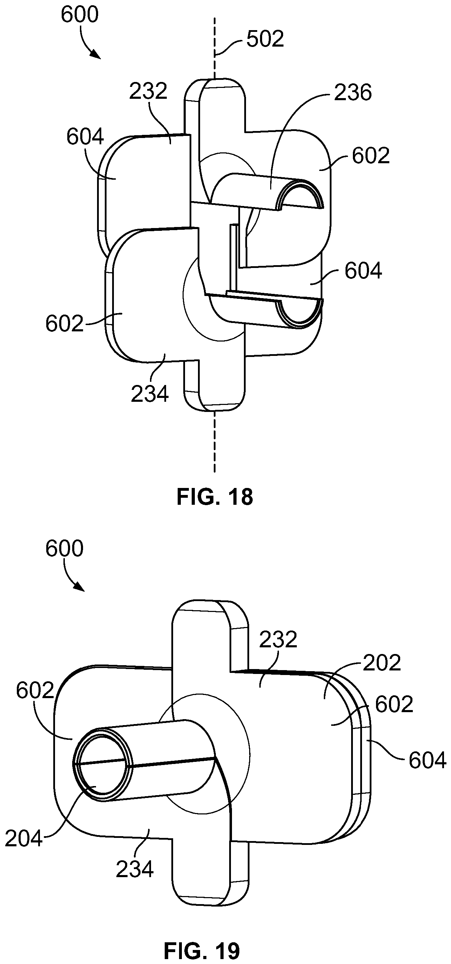

Figure 18 is a perspective view of a VBT 600 in the open state according to a fifth embodiment. Figure 19 is a perspective view of the VBT 600 of Figure 18 in the closed state. The VBT 600 is another two-piece, slidable matting design similar to the VBT 500 in Figures 16 and 17. The VBT 600 differs from the VBT 500 because instead of mating via at least one pin, the first section 232 of the VBT 600 and the discrete, second section 234 of the VBT 600 nest with each other to provide alignment and retain the closed state. For example, portions of the first section 232 overlap and contact portions of the second section 234, and the friction between the overlapping surfaces in contact may oppose forces that would cause the two sections 232, 234 to move apart and transition to the open state. The inherent retention in the nested sections may obviate including any additional retention elements, such as features dowel pins, magnets, or the like.

In an example, the first section 232 may include at least first and second flanges 602, 604 that extend outward from a proximal end of the curved member 236 that defines a portion of the tube 204. The first flange 602 may be offset from the second flange 604 along the depth direction, such that the first flange 602 is a front flange 602 that is located closer to the distal end of the curved member 236 than a proximity of the second, or back, flange 604 to the distal end of the curved member 236. The second section 234 may be a replica of the first section 232, such that the two parts have the same or a similar size, shape, and construction. During a mating process to transition from the open state to the closed state, the two sections 232, 234 may be moved relative to each other along the mating axis 502. The front flange 602 of the first section 232 may overlap in front of the back flange 604 of the second section 234. The back flange 604 of the first section 232 may overlap behind the front flange 602 of the second section 234. The overlapping flanges 602, 604 may contact each other, which retains the closed state via contact surface friction. When mated, the overlapping flanges 602, 604 define the back panel 202 of the VBT 600.

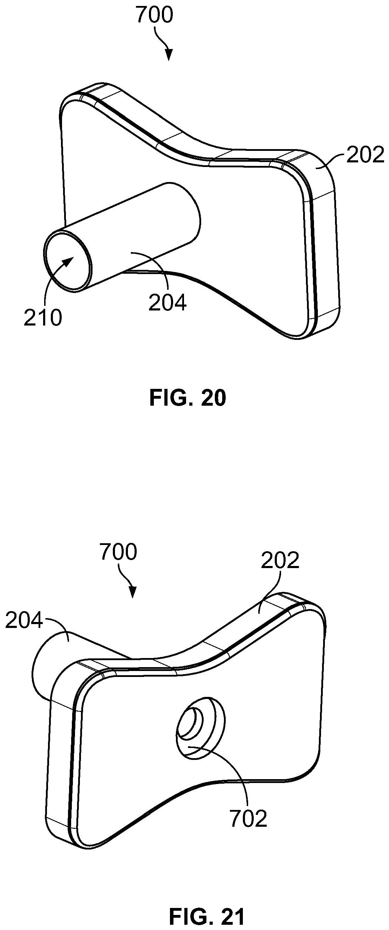

Figure 20 is a front perspective view of a VBT 700 according to a sixth embodiment. Figure 21 is a back perspective view of the VBT 700 of Figure 20. The VBT 700 differs from the above-described embodiments in that the tube 204 of the VBT 700 does not separate along any joint to remove the VBT 700 from the guide catheter 106. The VBT 700 is only removable in the traditional sense via sliding the VBT 700 past an end of the guide catheter 106. Thus, during a procedure, once the VBT 700 is pushed into position such that the tube 204 dilates the seal of the access introducer 110, the VBT 700 remains in that position until the guide catheter 106 is retracted out of the access introducer 110 and the VBT 700.

The VBT 700 has an integrated hemostasis seal 702. The seal 702 is integrated into the back panel 202. The seal 702 may be silicone or the like. In an example, the seal 702 may be secured in place between two parallel layers of the back panel 202. For example, the seal 702 may be within a stack of multiple layers that define the back panel 202. The seal 702 may be sized to contact and seal around the guide catheter 106. The seal 702 aligns with the channel 210 of the tube 204. Compared to the known VBT 2 that is shown in Figures 1 and 2, the VBT 700 has a significantly shorter length, which frees up more space along the delivery assembly 104. The VBT 700 may be significantly less complex than the VBT 2, as the VBT 700 avoids the elongated housing 3 and the side port 4.

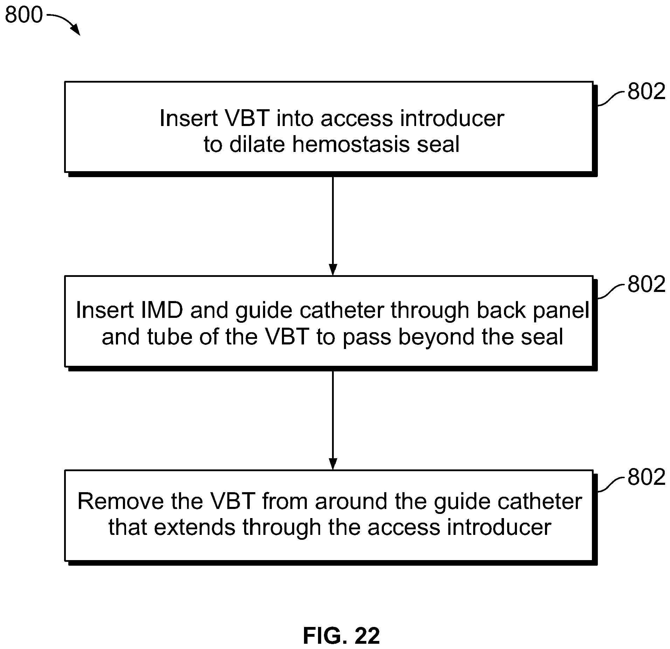

Figure 22 is a flow chart of a method 800 for assembling an IMD delivery system according to an embodiment. The method 800 may be performed using the delivery system 100 shown in Figure 100. The delivery system 100 includes a VBT. The VBT used to perform the method may be any of the VBTs 200, 300, 400, 500, 600, 700 of the embodiments described herein unless indicated otherwise. The method 800 optionally may include additional steps, fewer steps, and/or different steps than the steps shown and described in Figure 22.

At step 802, a VBT is inserted into a first inlet opening 290 of an access introducer 110. The VBT includes a back panel 202 and a tube 204 connected to the back panel 202 and extending from the back panel 202 to a distal end 208 of the tube 204. The tube 204 may be cylindrical for structural strength and rigidity. The tube 204 may define a channel 210 therethrough that aligns with and is open to a second inlet opening 212 defined through the back panel 202. Inserting the VBT may include advancing the tube 204 into the first inlet opening 290 such that the distal end 208 of the tube 204 dilates a seal of the access introducer 110.

At step 804, an IMD 152 and a guide catheter 106 are inserted through the second inlet opening 212 and the channel 210 of the VBT while the tube 204 is within the access introducer 110. The tube 204 holds the seal in the open, dilated state, which allows the IMD 152 and the guide catheter 106 to pass beyond the seal of the access introducer 110 towards a target implant location within a subject's anatomy. In an embodiment, the IMD 152 is leadless. For example, the leadless IMD may be a leadless pacemaker.

Optionally, at step 806, the VBT is removed from around the guide catheter 106 while a portion of the guide catheter 106 is located within the access introducer 110. For example, the back panel 202 and the tube 204 of the VBT are defined by at least a first section 232 and a second section 234. The tube 204 may be defined by at least a first curved member 236 of the first section 232 and a second curved member 240 of the second section 234. In one or more embodiments, removing the VBT includes pivoting the first section 232 relative to the second section 234 about a hinge to cause at least one gap 246 along a length of the tube 204 between the first curved member 236 and the second curved member 240. In a first embodiment, the hinge is a living hinge 230 that extends along the length of the tube 204 and extends from the first curved member 236 to the second curved member 240 to connect the two curved members 236, 240. In a second embodiment, the hinge is a mechanical hinge 302, 402. In one or more other embodiments, removing the VBT includes separating the first and second sections 232, 234 along a mating axis 502 to disconnect the first section 232 from the second section 234.

The method 800 may be performed during an implant procedure to advance an IMD to the target location, as described above, as well as during an extraction procedure to remove and withdraw an IMD from within the subject. The removable VBT may be removed from around the guide catheter 106 whenever the VBT is not necessary, and then reinstalled to surround the guide catheter 106 when the seal of the access introducer 110 needs to be dilated to move the guide catheter 106 relative to the access introducer 110.

The various VBTs described herein provide several benefits, such as interchangeability, reduced complexity, reduced cost, reduced interference with other delivery system components, and/or reduced size to free up additional length for providing longer delivery assemblies to reach farther into the subject or simply shorten the delivery system for a more compact product.

It should be clearly understood that the various arrangements and processes broadly described and illustrated with respect to the Figures, and/or one or more individual components or elements of such arrangements and/or one or more process operations associated of such processes, can be employed independently from or together with one or more other components, elements and/or process operations described and illustrated herein. Accordingly, while various arrangements and processes are broadly contemplated, described and illustrated herein, it should be understood that they are provided merely in illustrative and non-restrictive fashion, and furthermore can be regarded as but mere examples of possible working environments in which one or more arrangements or processes may function or operate.

It is to be understood that the subject matter described herein is not limited in its application to the details of construction and the arrangement of components set forth in the description herein or illustrated in the drawings hereof. The subject matter described herein is capable of other embodiments and of being practiced or of being carried out in various ways. Also, it is to be understood that the phraseology and terminology used herein is for the purpose of description and should not be regarded as limiting. The use of "including," "comprising," or "having" and variations thereof herein is meant to encompass the items listed thereafter and equivalents thereof as well as additional items.

It is to be understood that the above description is intended to be illustrative, and not restrictive. For example, the above-described embodiments (and/or aspects thereof) may be used in combination with each other. In addition, many modifications may be made to adapt a particular situation or material to the teachings herein without departing from its scope. While the dimensions, types of materials and coatings described herein are intended to define various parameters, they are by no means limiting and are illustrative in nature. Many other embodiments will be apparent to those of skill in the art upon reviewing the above description. The scope of the embodiments should, therefore, be determined with reference to the appended claims, along with the full scope of equivalents to which such claims are entitled. In the appended claims, the terms "including" and "in which" are used as the plain-English equivalents of the respective terms "comprising" and "wherein." Moreover, in the following claims, the terms "first," "second," and "third," etc. are used merely as labels, and are not intended to impose numerical requirements on their objects or order of execution on their acts.

## Claims

1. A valve bypass tool (200, 300, 400, 500, 600, 700) for an implantable medical device (IMD) delivery system (100), the valve bypass tool (200, 300, 400, 500, 600, 700) comprising:
a back panel (202) that defines an inlet opening (212); and
a tube (204) connected to the back panel (202) and extending from the back panel (202) to a distal end (208) of the tube (204), wherein the tube (204) is cylindrical and the distal end (208) of the tube (204) is configured to dilate a seal of an access introducer (110), the tube (204) defining a channel (210) therethrough that aligns with and is open to the inlet opening (212) in the back panel (202), wherein the inlet opening (212) and the channel (210) of the tube (204) are sized to receive an IMD (10, 152) therethrough.

2. The valve bypass tool of claim 1, wherein the inlet opening (212) and the channel (210) of the tube (204) are sized to receive a leadless IMD (10) as the IMD (10, 152).

3. The valve bypass tool of claim 1 or 2, wherein a diameter of the channel (210) of the tube (204) is 15-35 F (5-11.67 mm).

4. The valve bypass tool of any one of claims 1 to 3, wherein the back panel (202) is planar and includes a secondary seal configured to seal around a guide catheter (106) inserted through the valve bypass tool (200, 300, 400, 500, 600, 700).

5. The valve bypass tool of any one of claims 1 to 4, wherein the back panel (202) and the tube (204) are defined by at least a first section (232) and a second section (234), the first section (232) and the second section (234) selectively movable relative to each other to define a closed state of the valve bypass tool (200, 300, 400, 500, 600, 700) and an open state of the valve bypass tool, (200, 300, 400, 500, 600, 700) wherein in the open state a first curved member (236) of the first section (232) is spaced apart from a second curved member (240) of the second section (234) to define at least one gap (246) along a length of the tube (204).

6. The valve bypass tool of claim 5, wherein the at least one gap (246) in the open state is larger than a diameter of a guide catheter (106) inserted through the valve bypass tool (200, 300, 400, 500, 600, 700).

7. The valve bypass tool of claim 5 or 6, wherein the first section (232) is connected to the second section (234) via a living hinge (230), and the first and second sections (232, 234) are pivotable about the living hinge (230).

8. The valve bypass tool of claim 7, wherein the living hinge (230) extends along the length of the tube (204) and connects the first curved member (236) to the second curved member (240).

9. The valve bypass tool of claim 5 or 6, wherein the first section (232) is coupled to the second section (234) via a mechanical hinge (302), and the first and second sections (232, 234) are pivotable about the mechanical hinge (302).

10. The valve bypass tool of claim 9, wherein the back panel (202) is defined by at least a first flange member (238) of the first section (232) and a second flange member (242) of the second section (234), wherein the mechanical hinge (302) is secured to the first flange member (238) and the second flange member (242).

11. The valve bypass tool of claim 10, wherein the mechanical hinge (302) includes a pin (308) that projects through a first ear (310) of the first flange member (238) and a second ear (312) of the second flange member (242).

12. The valve bypass tool of claim 5 or 6, wherein the first section (232) and the second section (234) are movable relative to one another along a mating axis (502) to transition between the open state and the closed state.

13. The valve bypass tool of claim 12, wherein the first section (232) includes at least a first pin (506) and the second section (234) includes at least a first pinhole (508) that is sized and positioned to align with the first pin (506), such that the first pin (506) is received within the first pinhole (508) as the first and second sections (232, 234) are moved along the mating axis (502) to the closed state.

14. The valve bypass tool of claim 12 or 13, wherein the first section (232) includes a first front flange (602) and a first back flange (604) extending from the first curved member (236), and the second section (234) includes a second front flange (602) and a second back flange (604) extending from the second curved member (240), wherein the first front flange (602) overlaps and contacts the second back flange (604) and the first back flange (604) overlaps and contacts the second front flange (602) as the first and second sections (232, 234) are moved along the mating axis (502) to the closed state.

15. A method for assembling an implantable medical device (IMD) delivery system (100), the method comprising:
inserting a valve bypass tool (VBT) (200, 300, 400, 500, 600, 700) into a first inlet opening (290) of an access introducer (110), wherein the VBT (200, 300, 400, 500, 600, 700) includes a back panel (202) and a tube (204) connected to the back panel (202) and extending from the back panel (202) to a distal end (208) of the tube (204), wherein the tube (204) is cylindrical and defines a channel (210) therethrough that aligns with and is open to a second inlet opening (212) defined through the back panel (202), wherein inserting the VBT (200, 300, 400, 500, 600, 700) comprises advancing the tube (204) into the first inlet opening (290) such that the distal end (208) of the tube (204) dilates a seal of the access introducer (110); and
inserting an IMD (10, 152) and a guide catheter (106) through the second inlet opening (212) and the channel (210) of the VBT (200, 300, 400, 500, 600, 700) while the tube (204) is within the access introducer (110) such that the IMD (10, 152) and the guide catheter (106) pass beyond the seal of the access introducer (110).
